# EUROPEAN PATENT SPECIFICATION

(11) **EP 3 088 511 B1**
(45) Date of publication and mention of the grant of the patent: **30.12.2020**
(21) Application number: 14873584.8
(22) Date of filing: 19.08.2014
(51) Int. Cl.: G01N 15/14, C12M 1/34, G01N 21/27, G01N 33/48

(54) **IMAGE PROCESSING DEVICE, PROGRAM, INFORMATION STORAGE MEDIUM, AND IMAGE PROCESSING METHOD**
BILDVERARBEITUNGSVORRICHTUNG, PROGRAMM, INFORMATIONSSPEICHERMEDIUM UND BILDVERARBEITUNGSVERFAHREN
DISPOSITIF DE TRAITEMENT DE L'IMAGE, PROGRAMME, SUPPORT DE STOCKAGE DE L'INFORMATION, ET PROCÉDÉ DE TRAITEMENT DE L'IMAGE

(30) Priority: 27.12.2013 JP 2013271140
(43) Date of publication of application: 02.11.2016
(73) Proprietor: FUJIFILM Corporation, Tokyo, 106-8620 (JP)
(72) Inventor: OZAKI, RYOTA, Yokohama-shi Kanagawa 220-0012 (JP); ODA, HIDETO, Yokohama-shi Kanagawa 220-0012 (JP); KATO, NORIJI, Yokohama-shi Kanagawa 220-0012 (JP)
(74) Representative: Klunker IP Patentanwälte PartG mbB
(86) International application number: PCT/JP2014/071596
(87) International publication number: WO 2015/098177

(56) References cited:
- EP-A1- 1 414 231
- WO-A1-01/37012
- WO-A1-99/45385
- JP-A- 2002 214 228
- JP-A- 2003 504 627
- JP-A- 2010 216 920
- JP-A- 2011 177 063
- JP-A- 2013 027 368
- US-A1- 2002 010 618
- US-A1- 2008 241 848
- US-A1- 2012 314 092

## Description

### Technical Field

The present invention relates to an image processing apparatus, a program, an information storage medium, and an image processing method.

### Background Art

For example, in PTL 1, maternal blood serves as a specimen, and nucleated red blood cells in the maternal blood serve as target cells. On the basis of a nucleus candidate region extracted from a microscopy image of the specimen, a determination target region is set, and whether target cells are included in the set determination target region is determined.

PTL 2 discloses a method of finding a predetermined number of fetal cells in a maternal blood sample by screening smears until at least one smear with the predetermined number of fetal cells is found. This smear is then subjected to processing for diagnostic purposes.

PTL 3 discloses an automated system for analyzing samples containing cells. A first digital image is captured of a first viewable portion of a sample containing cells to be counted, and the image is analyzed to count the number of cells. This count is compared with a first threshold chosen to distinguish tests where analysis of a single image is sufficient to obtain a satisfactory concentration estimate and tests where multiple images are needed. If the count obtained from analysis of the first digital image exceeds the first threshold, a test result is reported; otherwise a second digital image is captured of a second viewable portion of the sample, and analyzed to determine a cell count. The combined count of cells detected in the first and second images is compared with a second threshold and if this threshold is exceeded, a test result is reported, otherwise a third viewable portion is found.

PTL 4 discloses an imager for imaging a sample comprising an imager stage that supports the sample, a fiber optic bundle having a first bundle end of first fiber ends arranged to define an input aperture viewing the sample on the imager stage, and a second bundle end of second fiber ends arranged to define an output aperture shaped differently from the input aperture and disposed away from the imager stage, a scanning radiation source arranged to scan a radiation beam on the sample within a viewing area of the input aperture, the radiation beam interacting with the sample to produce a light signal that is received by the input aperture and transmitted via the fiber optic bundle to the output aperture, a photodetector arranged to detect the light signal at the second bundle end, and a processor that processes the light signal detected by the photodetector.

### Citation List

### Patent Literature

PTL 1: Japanese Unexamined Patent Application Publication No. 2012-254042
PTL 2: US2008/241848 A1
PTL 3: US2012/314092 A1
PTL 4: EP 1 414 231 A1

### Summary of Invention

### Technical Problem

It is an object of the present invention to provide an image processing apparatus, a computer program, a computer-readable storage medium, and an image processing method capable of setting parameters of a process of detecting target cells in a target specimen, in accordance with a feature of the target specimen.

### Solution to Problem

This object is achieved by the features of the respective independent claims. Further embodiments are defined in the corresponding dependent claims.

### Advantageous Effects of Invention

Accordingly, parameters of a process of detecting target cells in a target specimen can be set in accordance with a feature of the target specimen, and the number of samples serving as processing targets, among a plurality of samples generated from a target specimen, and a determination target region set for each sample can be set in accordance with the estimated number of target cells, which is estimated for the target specimen.

Accordingly, selection can be made so as to include target cells, the number of which is greater than or equal to a threshold, in a sample serving as a processing target.

Accordingly, when the difference between the number of target cells included in a sample serving as a processing target and the threshold is small, the number of regions serving as determination targets is increased, thereby preventing the occurrence of detection evasion.

Accordingly, parameters of a process of detecting target cells in a target specimen can be set in accordance with the difficulty in detecting target cells in a specimen serving as a processing target.

Accordingly, when the difficulty in detecting target cells in a specimen serving as a processing target is high, the number of regions serving as determination targets is increased, thereby preventing the occurrence of detection evasion.

Accordingly, the difficulty in detecting target cells in a specimen serving as a processing target can be accurately calculated, compared with the case where the present configuration is not provided.

Accordingly, an accurate determination result based on the determination results of a plurality of determination target regions for the same target can be obtained, compared with the case of not integrating a plurality of determination target region.

Accordingly, the number of nucleated red blood cells included in target maternal blood can be accurately estimated, compared with the case where the present configuration is not provided.

### Brief Description of Drawings

[Fig. 1] Fig. 1 is a diagram illustrating an exemplary system configuration of an image processing system according to an embodiment.
[Fig. 2] Fig. 2 is a functional block diagram illustrating an example of functions included in an image processing apparatus.
[Fig. 3] Fig. 3 is a diagram illustrating an example of a detection parameter management table.
[Fig. 4] Fig. 4 is a diagram describing exemplary integration of target cell candidate regions.
[Fig. 5] Fig. 5 is a flowchart of a process according to a first example.
[Fig. 6] Fig. 6 is a flowchart of a process of calculating the detection difficulty.
[Fig. 7] Fig. 7 is a flowchart of a process of detecting target cells.
[Fig. 8] Fig. 8 is a flowchart of a process of integrating target cell regions.
[Fig. 9] Fig. 9 is a flowchart of a process according to a second example.
[Fig. 10] Fig. 10 is a flowchart of a process according to a third example.

### Description of Embodiments

### [1. Description of System Configuration]

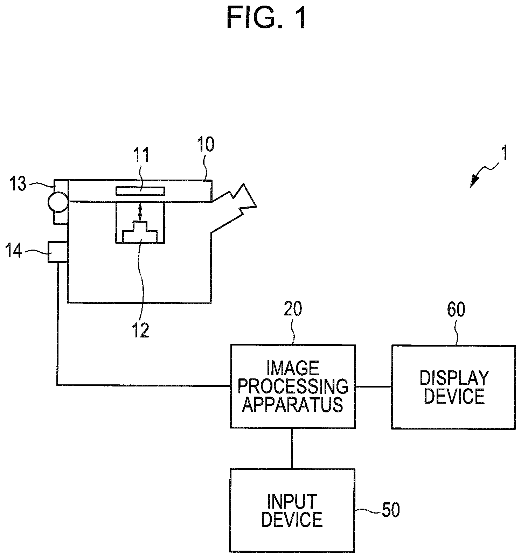

Fig. 1 illustrates an exemplary system configuration of an image processing system 1 according to the embodiment. As illustrated in Fig. 1, the image processing system 1 includes an optical microscope 10, an image processing apparatus 20, an input device 50, and a display device 60. The image processing apparatus 20 is connected to the optical microscope 10, the input device 50, and the display device 60 so as to be capable of communicating data.

The optical microscope 10 captures, with a CCD camera 14, an image of a preparation on a slide 11 placed on a stage, via an optical system such as an objective lens(es) 12. The optical microscope 10 includes a focusing mechanism 13 that changes the distance between the slide 11 and the objective lens 12, and the optical microscope 10 is configured to capture an image of the preparation on the slide 11 at different focusing distances. In the embodiment, maternal blood is applied to the slide 11, and the slide 11 is stained with May-Giemsa, which is used as a preparation. This stains fetal nucleated red blood cells (NRBCs) in the maternal blood blue-purple. Hereinafter, NRBCs are referred to as target cells.

The image processing apparatus 20 obtains a captured image, captured by the optical microscope 10, and detects target cells in the obtained captured image. For example, the image processing apparatus 20 may determine a score (such as a probability) indicating the probability that target cells are included in a determination target region set in the captured image, captured by the optical microscope 10, on the basis of a discriminator that has learned conditions for discriminating target cells. Note that a process of detecting target cells, performed by the image processing apparatus 20, will be described in detail later.

The input device 50 is a device such as a keyboard or a mouse, and inputs an operation accepted from a user to the image processing apparatus 20. For example, the image processing apparatus 20 obtains information on an image region specified, regarding an image displayed on the display device 60, by the user by using the input device 50 as learning information for learning positive and negative examples of target cells, or image features of other particular cells, and may have the discriminator learn conditions (discrimination parameters) for discriminating target cells on the basis of the learning information.

The display device 60 is, for example, a liquid crystal display device 60, and displays a screen on the basis of the result of a process performed by the image processing apparatus 20. For example, the display device 60 displays a captured image captured by the optical microscope 10, the result of detecting target cells by the image processing apparatus 20, or the like.

### [2. Description of Functions Included in Image Processing Apparatus 20]

Next, functions included in the image processing apparatus 20 according to the embodiment will be described.

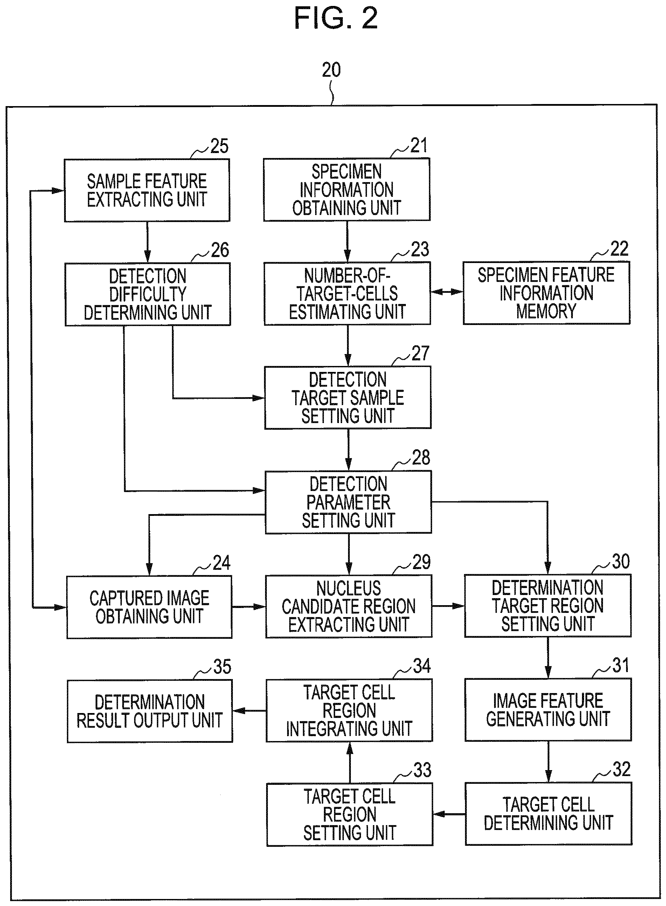

Fig. 2 is a functional block diagram illustrating an example of functions included in the image processing apparatus 20. As illustrated in Fig. 2, the image processing apparatus 20 includes a specimen information obtaining unit 21, a specimen feature information memory 22, a number-of-target-cells estimating unit 23, a captured image obtaining unit 24, a sample feature extracting unit 25, a detection difficulty determining unit 26, a detection target sample setting unit 27, a detection parameter setting unit 28, a nucleus candidate region extracting unit 29, a determination target region setting unit 30, an image feature generating unit 31, a target cell determining unit 32, a target cell region setting unit 33, a target cell region integrating unit 34, and a determination result output unit 35.

The functions of the above-mentioned units included in the image processing apparatus 20 may be realized by reading and executing a program stored in a computer-readable information storage medium, by the image processing apparatus 20, which is a computer including control means such as a CPU, storage means such as a memory, and input-output means that transmits/receives data to/from an external device. Note that the program may be supplied to the image processing apparatus 20 through an information storage medium such as an optical disk, a magnetic disk, a magnetic tape, a magneto-optical disk, or a flash memory, or may be supplied to the image processing apparatus 20 via a data communication network such as the Internet. Hereinafter, the functions of the units included in the image processing apparatus 20 will be described in detail.

The specimen information obtaining unit 21 obtains information on a specimen (mother) serving as a test target (specimen information). For example, the specimen information obtaining unit 21 may obtain specimen information on the basis of data input via the input device 50. For example, the specimen information may include the age, medical history, weeks of pregnancy, or the like of a person who is a test object from which maternal blood has been taken.

The specimen feature information memory 22 stores information for use in estimating target cells included in a specimen, on the basis of a feature of the specimen. For example, with respect to a category in accordance with a feature of a specimen, the specimen feature information memory 22 may store the number of target cells per unit blood volume according to each category, for example. For example, the image processing apparatus 20 may generate and cluster a feature vector for each specimen on the basis of each item of information, that is, the age, medical history, and weeks of pregnancy, and may associatively store a pre-measured representative value (such as an average value) of the number of target cells (nucleated red blood cells) per unit blood volume for a specimen belonging to each cluster, in the specimen feature information memory 22. In addition, the specimen feature information memory 22 may store a table or an equation defining the relationship between the age, medical history, and weeks of pregnancy with a corresponding number of nucleated red blood cells.

The number-of-target-cells estimating unit 23 estimates the number of target cells included in a specimen, on the basis of specimen information (information representing a feature of the specimen) regarding the specimen, obtained by the specimen information obtaining unit 21, and information stored in the specimen feature information memory 22. For example, for a category corresponding to the specimen information obtained by the specimen information obtaining unit 21, the number-of-target-cells estimating unit 23 estimates the number of target cells included in a preparation (maternal blood) obtained from the specimen, on the basis of a representative number of target cells, stored in the specimen feature information memory 22. For example, for a category corresponding to the specimen information obtained by the specimen information obtaining unit 21, the number-of-target-cells estimating unit 23 may obtain the estimated number of target cells by multiplying the number of nucleated red blood cells per unit blood volume, stored in the specimen feature information memory 22, and the blood volume of the preparation.

In addition, the method of estimating the number of target cells is not limited to that described above. For example, the specimen feature information memory 22 may store in advance, for one or more specimens, a feature vector(s) of the specimen(s) and the number of target cells in association with each another. The number-of-target-cells estimating unit 23 identifies a feature vector that is similar to a feature vector of a target specimen (for example, the distance between the feature vectors is shortest) among the feature vector(s) of the specimen(s), stored in the specimen feature information memory 22, and may regard the number of target cells associated with the identified feature vector as the number of target cells in the target specimen.

The captured image obtaining unit 24 obtains a captured image of a preparation (maternal blood) on the slide, obtained from a specimen. For example, the captured image obtaining unit 24 obtains, from the optical microscope 10, a captured image of a preparation (maternal blood), captured with the CCD camera 14 included in the optical microscope 102. In addition, the captured image obtaining unit 24 may obtain a captured image in the case where a detection target flag defined for a target preparation is true (T) on the basis of later-described detection parameters, and may not obtain a captured image in the case where a detection target flag defined for a target preparation is false (F).

The sample feature extracting unit 25 extracts features of a target sample. Features of a sample include information indicating a generation state of the sample. For example, the sample feature extracting unit 25 may extract, as sample features, the thickness evenness of a sample generated from a specimen, the staining intensity of the cell nucleus, the content by percentage of white blood cells, and the like. The sample feature extracting unit 25 may extract sample features of each of all or some of samples (such that samples are limited to those serving as detection targets) generated from a target specimen.

The sample feature extracting unit 25 may calculate, for example, the thickness evenness (A) of a sample by measuring the depth from the surface of the sample to the slide at a plurality of points in the sample by using the optical microscope 10, and obtaining the reciprocal of a variance of the measured depths.

The sample feature extracting unit 25 may calculate, for example, the staining intensity (B) of the cell nucleus in a sample as a value obtained by dividing a predetermined threshold by the average intensity value of a captured image of the sample (may be captured with the optical microscope 10) (that is, threshold/average intensity value), or as the proportion of pixels with intensity values lower than a predetermined threshold in a captured image of the sample.

The sample feature extracting unit 25 may calculate, for example, the content by percentage (C) of white blood cells in a sample as the proportion of pixels of colors that are lighter than a predetermined density in a captured image of the sample (may be captured with the optical microscope 10).

The detection difficulty determining unit 26 determines the difficulty (Df) in detecting target cells in a sample, on the basis of sample features extracted by the sample feature extracting unit 25. For example, the detection difficulty determining unit 26 may calculate the detection difficulty Df of a sample by using the following equation (1) on the basis of the thickness evenness A of the sample, the staining intensity B of the cell nucleus in the sample, and the content by percentage C of white blood cells in the sample, which are extracted by the sample feature extracting unit 25. Note that w1, w2, and w3 may be predetermined coefficients that satisfy w1+w2+w3=1 and that are greater than or equal to 0; A0 may be a reference value of the thickness evenness of the sample; B0 may be a reference value of the staining intensity of the cell nucleus in the sample; and C0 may be a reference value of the content by percentage of white blood cells in the sample. $Df = w 1 ⋅ A 0 / A + w 2 ⋅ B 0 / B + w 3 ⋅ C / C 0$

According to the above-mentioned equation (1), the detection difficulty Df is calculated to be lower as the value of the thickness evenness A of the sample becomes greater; the detection difficulty Df is calculated to be lower as the value of the staining intensity B of the cell nucleus in the sample becomes greater; and the detection difficulty Df is calculated to be greater as the value of the content by percentage C of white blood cells in the sample becomes greater. Note that the detection difficulty determining unit 26 may correct the detection difficulty Df, calculated by the above-mentioned equation (1), to take a value from 0 to 1. For example, the detection difficulty determining unit 26 may have 0 for Df when Df is less than a lower threshold and may have 1 for Df when Df is greater than an upper threshold (such as a value greater than 1).

The detection target sample setting unit 27 sets samples serving as detection targets from among a plurality of samples generated from a target specimen, on the basis of at least one of the estimated number (Cb) of target cells estimated by the number-of-target-cells estimating unit 23 and the detection difficulty (Df) determined by the detection difficulty determining unit 26.

The detection target sample setting unit 27 calculates the number of target cells per sample (a=Cb/N) by, for example, dividing the estimated number (Cb) of target cells estimated for a target specimen by the number (N) of samples generated from the preparation. Here, when Cb>Z for the necessary number (Z) of target cells, the detection target sample setting unit 27 determines an integer X that satisfies a·X≥Z as the number of samples serving as detection targets, and, when Cb<Z, the detection target sample setting unit 27 determines the number of samples serving as detection targets as N (all samples). The detection target sample setting unit 27 may select, from among N samples generated from the preparation, samples the number of which is equivalent to the number of samples serving as detection targets (for example, in the order of identification number or in the order of generation date and time), and may set the detection target flags of the selected samples to true (T) and the detection target flags of unselected samples to false (F).

In addition, in the case of setting samples serving as detection targets on the basis of the detection difficulty calculated by the detection difficulty determining unit 26, the detection target sample setting unit 27 may set, for example, among a plurality of samples generated from a target specimen, samples whose detection difficulties are less than or equal to a threshold as detection targets.

The detection parameter setting unit 28 sets detection parameters for use in a detection process for samples generated from a preparation. For example, the detection parameter setting unit 28 sets detection parameters on the basis of at least one of detection target samples set by the detection target sample setting unit 27 and the detection difficulty determined by the detection difficulty determining unit 26. For example, the detection parameters may include a detection target flag indicating whether the sample serves as a detection target, nucleus candidate region parameters indicating conditions of an image region extracted as a nucleus candidate region from a captured image of the sample, and determination target region parameters indicating conditions for setting a determination target region set for a captured image of the sample.

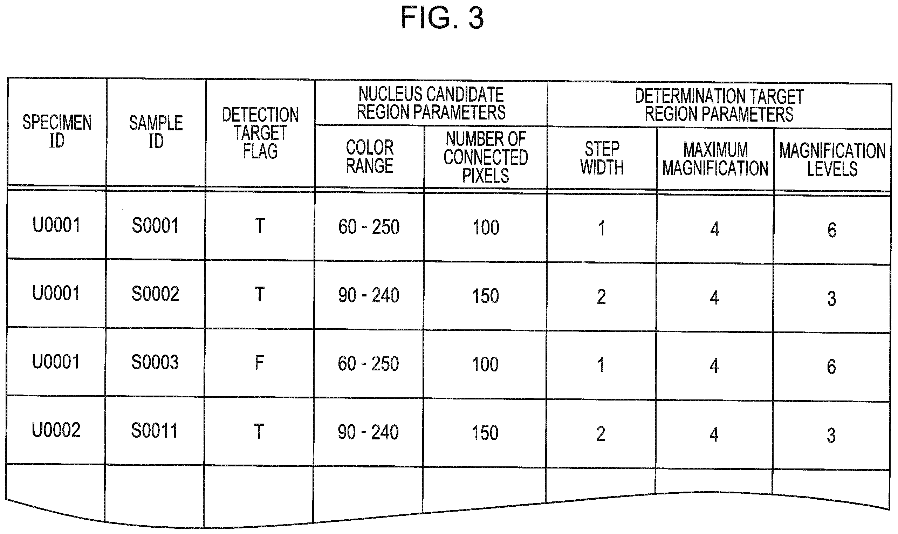

Fig. 3 illustrates an example of a detection parameter management table storing detection parameters set by the detection parameter setting unit 28. As illustrated in Fig. 3, the detection parameter management table associatively stores a specimen ID for identifying a specimen, a sample ID for identifying each of a plurality of samples obtained from the specimen, a detection target flag indicating whether each sample serves as a detection target, nucleus candidate region parameters, and determination target region parameters. Note that the nucleus candidate region parameters include the color range of target pixels extracted as a nucleus candidate region, and a threshold of the number of connected pixels. In addition, the determination target region parameters include a step width indicating the amount of shift between pixels serving as the base points of a determination target region in a nucleus candidate region, the maximum magnification indicating, regarding determination target regions of a plurality of sizes set for pixels serving as the base points of the determination target regions, a size ratio between the minimum determination target region and the maximum determination target region, and magnification levels indicating the number of levels of magnification executed from the minimum determination target region to the maximum determination target region.

The detection parameter setting unit 28 may set detection parameters on the basis of at least one of a margin Y, which is the difference between the product of the number X of samples serving as detection targets and the number a of target cells per sample, set by the detection target sample setting unit 27, and the necessary number Z of target cells (Y=a·X-Z), and the detection difficulty Df determined by the detection difficulty determining unit 26. More specifically, the detection parameter setting unit 28 may preliminarily determine detection parameters for each of M levels (L1 to LM) (M is an integer greater than or equal to 2) (note that, for the same image, the number of nucleus candidate regions and determination target regions extracted/set in accordance with detection parameters at Li+1 is greater than the number of nucleus candidate regions and determination target regions extracted/set in accordance with detection parameters at Li), determine the level on the basis of the value of at least one of the margin Y and the detection difficulty Df, and set detection parameters on the basis of the determined level. For example, the detection parameter setting unit 28 may preliminarily define the range of the margin for each of M levels, and may determine the level on the basis of to which level's range the above-calculated Y belongs. Alternatively, for example, the detection parameter setting unit 28 may preliminarily define the range of the detection difficulty for each of M levels, and may determine the level on the basis of to which level's range the above-calculated Df belongs. Alternatively, for example, the detection parameter setting unit 28 may preliminarily define the range of the sum of the margin and the detection difficulty for each of M levels, and may determine the level on the basis of to which level's range the above-calculated sum of Y and Df belongs.

Note that the detection parameter setting unit 28 may set, for each sample set by the detection target sample setting unit 27, detection parameters that are different for each sample on the basis of the detection difficulty calculated for each sample.

The nucleus candidate region extracting unit 29 extracts, from a captured image, obtained by the captured image obtaining unit 24, of a sample serving as a processing target, a nucleus candidate region on the basis of nucleus candidate region parameters set by the detection parameter setting unit 28 for the sample. For example, the nucleus candidate region extracting unit 29 may perform binarization by regarding pixels included in a color range included in the nucleus candidate region parameters as black pixels and pixels not included in the color range as white pixels, and, among connected pixel groups of connected adjacent black pixels, may extract, as a nucleus candidate region, a connected pixel group having connected pixels whose number is greater than or equal to the number of connected pixels included in the nucleus candidate region parameters.

The determination target region setting unit 30 sets, for a captured image, obtained by the captured image obtaining unit 24, of a sample serving as a processing target, a determination target region subjected to determination of whether there are target cells, on the basis of determination target region parameters set by the detection parameter setting unit 28 for the sample and a nucleus candidate region extracted by the nucleus candidate region extracting unit 29. For example, the determination target region setting unit 30 sets, for each of one or more pixels included in a nucleus candidate region extracted by the nucleus candidate region extracting unit 29, a rectangular region around that pixel (or with reference to that pixel, which serves as a base point), as a determination target region. Here, the determination target region setting unit 30 may sequentially set determination target regions by sequentially moving, on the basis of a step width (shift amount) included in determination target region parameters set for a sample serving as a processing target, a pixel that is in a nucleus candidate region and that serves as the base point of a determination target region, by the step width. In addition, the determination target region setting unit 30 may set, regarding a pixel serving as the base point of a determination target region, determination target regions of different sizes by changing, on the basis of the maximum magnification and the magnification levels included in the determination target region parameters set for a sample serving as a processing target, the size of a determination target region from 1 to the maximum magnification, over the number of levels defined by the magnification levels.

The image feature generating unit 31 generates an image feature amount of a determination target region set by the determination target region setting unit 30. For example, the image feature generating unit 31 may calculate an HOG feature amount of a determination target region, and obtain the HOG feature amount as an image feature amount. Hereinafter, a process of calculating two types of HOG feature amounts will be specifically described.

The image feature generating unit 31 obtains the intensity gradient orientation and the intensity gradient magnitude at each pixel in a determination target region, splits a target image into Y blocks each having X cells, obtains a histogram of oriented gradients ([a first gradient orientation value, a second gradient orientation value, ..., and a ninth gradient orientation value]) from the intensity gradient orientations and the intensity gradient magnitudes for each cell included in each block, and performs normalization in units of blocks so that the mean square thereof becomes 1. The image feature generating unit 31 obtains X×9, which is a value generated by combining the above-mentioned normalized histograms of oriented gradients in each block, as a feature value of that block, and obtains Y×X×9, which is a value generated by combining all the blocks in the target image, as an HOG feature amount of the determination target region.

In addition, the image feature generating unit 31 obtains the intensity gradient orientation and the intensity gradient magnitude at each pixel in a determination target region, splits a target image into Y blocks each having X cells, and obtains a histogram of oriented gradients ([a first gradient orientation value, a second gradient orientation value, ..., and an eighteenth gradient orientation value]) from the intensity gradient orientations and the intensity gradient magnitudes for each cell included in each block. The image feature generating unit 31 obtains X×18, which is a value generated by combining the above-mentioned histograms of oriented gradients in each block, as a feature value of that block, and obtains Y×X×18, which is a value generated by combining all the blocks in the target image, as a Cell-HOG feature amount of the determination target region.

The target cell determining unit 32 determines, on the basis of the image feature amount of a determination target region, the probability (reliability) that target cells are included in the determination target region. For example, a discriminator may be caused in advance to learn conditions for discriminating target cells (discrimination parameters) on the basis of the image feature amount of an image region where target cells are shown, and the result of discriminating the image feature amount of a determination target region, obtained by the discriminator, may be obtained. Note that AdaBoost, SVM (support vector machine), or the like may be used as the discriminator. The discriminator outputs, on the basis of the image feature amount of a determination target region, a score indicating the probability (reliability) that target cells are included in the determination target region. For example, the discriminator may output a score having a positive value when cells included in a determination target region are target cells, and may output a score having a negative value when the cells are not target cells.

On the basis of the determination result obtained by the target cell determining unit 32 for a determination target region, the target cell region setting unit 33 sets a candidate region (target cell region) including target cells. For example, the target cell region setting unit 33 may set a determination target region as a target cell candidate region when the reliability output by the discriminator is greater than or equal to 0.

The target cell region integrating unit 34 integrates, among candidate regions set by the target cell region setting unit 33, candidate regions that at least partially overlap each other into one region. For example, when a plurality of candidate regions overlap each other, the target cell region integrating unit 34 may integrate the plurality of candidate regions into one of these regions (a region with the maximum reliability, for example). Here, the target cell region integrating unit 34 may determine that a plurality of candidate regions overlap each other when these regions are determination target regions set from the same nucleus candidate region, or may determine that a plurality of candidate regions overlap each other when the plurality of candidate regions have an overlapping portion whose area is greater than or equal to a predetermined threshold. In addition, regarding the integrated candidate regions, the target cell region integrating unit 34 may leave only a candidate region with the highest reliability, or may have a region including all the overlapping candidate regions as an integrated candidate region. In addition, the target cell region integrating unit 34 may obtain the reliability of an integrated candidate region by adding the reliabilities of the candidate regions that have been integrated, or may obtain the reliability of an integrated candidate region by multiplying that reliability by the number of overlapping candidate regions.

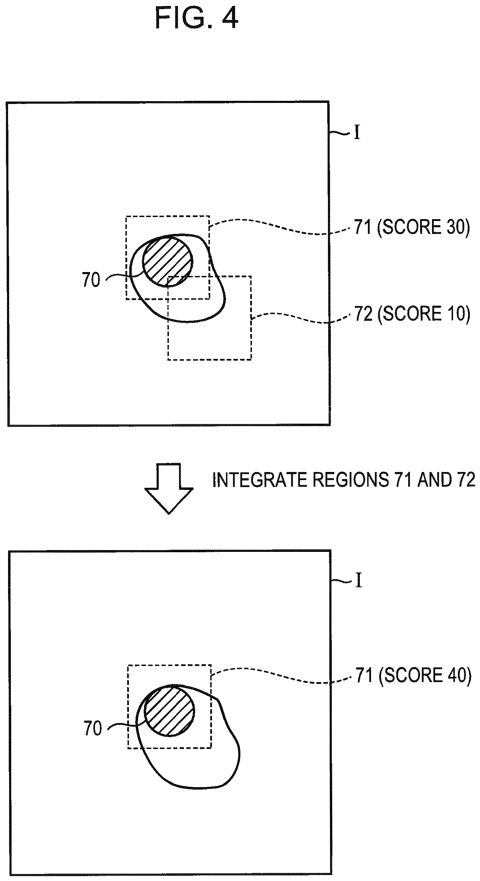

Fig. 4 is a diagram describing exemplary integration of target cell candidate regions. As illustrated in Fig. 4, a captured image I includes a nucleus candidate region 70. For the nucleus candidate region 70, two target cell regions 71 and 72, which partially overlap each other, are set. If the score for the target cell region 71 is 30 and the score for the target cell region 72 is 10, the target cell region integrating unit 34 may integrate the target cell region 72 with the target cell region 71 whose score is maximum out of the target cell regions 71 and 72, add the score of the target cell region 72 to the score of the target cell region 71, and update the score.

The determination result output unit 35 outputs information on candidate regions integrated by the target cell region integrating unit 34 as a target cell detection result for a preparation. For example, the determination result output unit 35 may display a display screen displaying candidate regions integrated by the target cell region integrating unit 34, which are sorted according to the reliability and displayed in a list, on the display device 60. Note that the determination result output unit 35 may not include a candidate region whose reliability is less than a specified or predetermined threshold in the displayed list.

### [3. Exemplary Process]

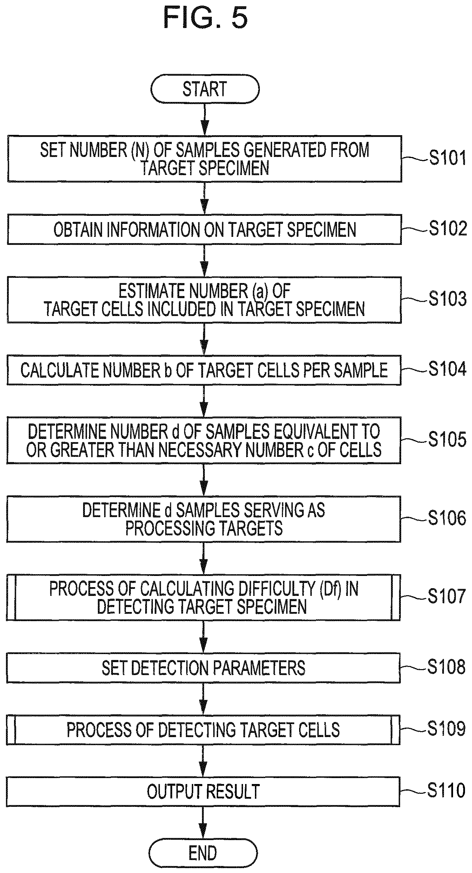

Next, an exemplary process performed in the image processing system 1 according to the embodiment will be described in detail on the basis of Figs. 5 to 10.

### [3-1. First Example]

First, a process according to a first example, executed by the image processing system 1, will be described in detail on the basis of the flowcharts illustrated in Figs. 5 to 8.

### [3-1-1(1). Main Process (1)]

As illustrated in Fig. 5, on the basis of data input from the input device 50, the image processing apparatus 20 sets the number (N (N is an integer greater than or equal to 1)) of samples generated from a target specimen (S101), and obtains information on the target specimen (specimen information) (S102). Here, the specimen information may include at least one of information on the age, medical history, and weeks of pregnancy.

Next, on the basis of the specimen information obtained in S102, the image processing apparatus 20 estimates the number (a) of target cells included in the target specimen (S103). For example, the image processing apparatus 20 may identify a category to which the specimen information obtained in S102 belongs, and, on the basis of a representative number of target cells (a reference number of target cells) per unit blood, which is predetermined for the identified category, and the volume of blood of a preparation serving as the specimen, may calculate the estimated number of target cells included in the specimen.

On the basis of the number (a) of target cells, calculated in S103, and the number (N) of samples, set in S101, the image processing apparatus 20 calculates the number (b) of target cells per sample (S104). On the basis of the number (b) of target cells per sample, calculated in S104, the image processing apparatus 20 determines the number (d) of detection target samples equivalent to or greater than a necessary number (c) of cells needed for detection (S105).

Here, the image processing apparatus 20 selects and determines samples serving as processing targets, the number of which is the number (d) of detection target samples, determined in S105, from among a plurality of samples generated from the target specimen (S106).

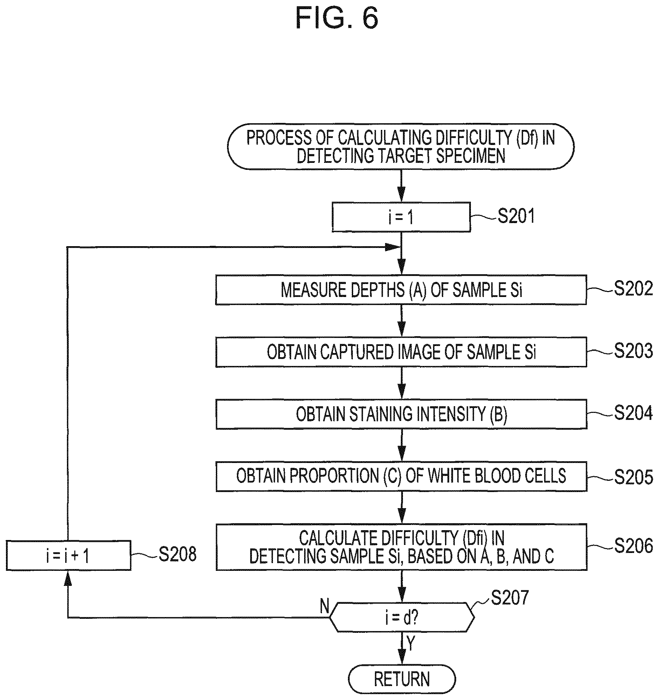

Next, the image processing apparatus 20 executes a process of calculating the detection difficulty indicating the difficulty in detecting target cells in each sample serving as a processing target, determined in S106 (S107). The process of calculating the detection difficulty will be described in detail on the basis of the flowchart illustrated in Fig. 6.

### [3-1-2. Process of Calculating Detection Difficulty]

As illustrated in Fig. 6, the image processing apparatus 20 initializes a variable i to 1 (S201), and measures depths (A) of a sample Si (S202). For example, the depth from the surface of the sample Si to the slide may be measured at a plurality of points in the sample Si by using the optical microscope 10.

Next, the image processing apparatus 20 obtains a captured image of the sample Si, captured using the optical microscope 10 (S203), and obtains the staining intensity (B) of the sample Si on the basis of the obtained captured image (S204). For example, the image processing apparatus 20 may calculate the staining intensity (B) of the cell nucleus in the sample Si as a value obtained by dividing a predetermined threshold by the average intensity value of the captured image of the sample Si (that is, threshold/average intensity value), or as the proportion of pixels with intensity values lower than a predetermined threshold in the captured image of the sample.

Further, on the basis of the captured image obtained in S203, the image processing apparatus 20 obtains the proportion (C) of white blood cells in the sample Si (S205). For example, the image processing apparatus 20 may calculate the proportion (C) of white blood cells in the sample Si as the proportion of pixels of colors lighter than a predetermined density in the captured image of the sample Si.

The image processing apparatus 20 calculates the detection difficulty Df (Dfi) in detecting target cells in the sample Si, on the basis of, for example, the above-described equation (1) and on the basis of the variance of the depths (A), the staining intensity (B) of the preparation, and the proportion (C) of white blood cells (S206).

Here, when the variable i has not reached d (S207: N), the image processing apparatus 20 increments the variable i (adds 1) (S208) and returns to S202; and, when the variable i has reached d (S207: Y), the image processing apparatus 20 returns. Next, referring back to the flowchart in Fig. 5, a description will be continuously given.

### [3-1-1(2). Main Process (2)]

As illustrated in Fig. 5, having finished calculating the detection difficulty Df in detecting target cells in a sample serving as a processing target, the image processing apparatus 20 sets detection parameters on the basis of at least one of the detection difficulty Df, calculated in S107, and the information on each sample serving as a processing target, determined in S106 (S108). For example, the detection parameters may include a detection target flag indicating whether the sample serves as a detection target, nucleus candidate region parameters indicating conditions of an image region extracted as a nucleus candidate region from the sample, and determination target region parameters indicating conditions for setting a determination target region set for an image of the sample. Here, the image processing apparatus 20 may set, for example, the detection target flag on the basis of the information on each sample serving as a processing target, determined in S106, and may set the nucleus candidate region parameters and the determination target region parameters on the basis of the detection difficulty Dfi calculated for the sample Si.

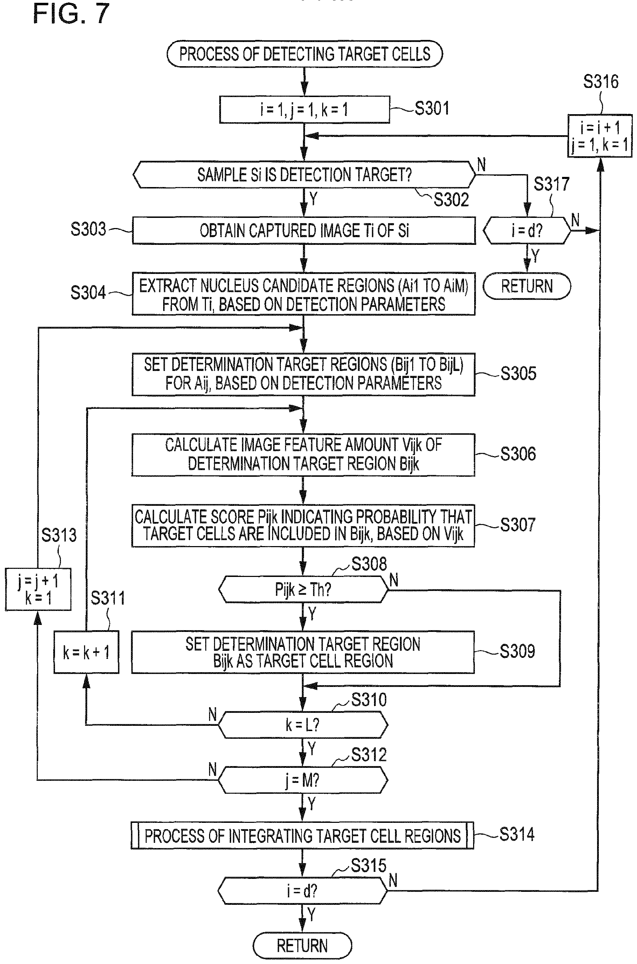

On the basis of the detection parameters set in S108, the image processing apparatus 20 executes a process of detecting target cells in the specimen (S109). The process of detecting target cells will be described in detail on the basis of the flowchart illustrated in Fig. 7.

### [3-1-3. Process of Detecting Target Cells]

As illustrated in Fig. 7, the image processing apparatus 20 initializes the values of variables i, j, and k to 1 (S301), and determines whether a sample Si of a target specimen is a detection target (S302). For example, the image processing apparatus 20 may determine that the sample Si is a detection target when the detection target flag of the sample Si is true (T), and may determine that the sample Si is not a detection target when the detection target flag is false (F).

When the sample Si is a detection target (S302: Y), the image processing apparatus 20 causes, for example, the optical microscope 10 to capture an image of the sample Si, and obtains a captured image Ti of the sample Si (S303).

Next, the image processing apparatus 20 extracts nucleus candidate regions (Ai1 to AiM) from the captured image Ti, on the basis of the nucleus candidate region parameters included in the detection parameters set for the sample Si (S304). Note that M is the number of nucleus candidate regions included in the captured image Ti. Note that extraction of nucleus candidate regions may be executed by the above-described nucleus candidate region extracting unit 29.

The image processing apparatus 20 sets determination target regions (Bij1 to BijL) for a nucleus candidate region Aij extracted from the captured image Ti, on the basis of the determination target region parameters included in the detection parameters set for the sample Si (S305). Note that L is the number of determination target regions set for the nucleus candidate region Aij, and setting of determination target regions may be executed by the above-described determination target region setting unit 30.

The image processing apparatus 20 calculates an image feature amount Vijk of a determination target region Bijk (S306). Note that calculation of an image feature amount may be executed by the above-described image feature generating unit 31.

On the basis of the image feature amount Vijk calculated in S306, the image processing apparatus 20 calculates a score Pijk indicating the probability that target cells are included in the determination target region Bijk (S307).

Here, when the score Pijk calculated in S307 is greater than or equal to a threshold Th (S308: Y), the image processing apparatus 20 sets the determination target region Bijk as a target cell region (S309).

After S309 or when the score Pijk calculated in S307 is less than the threshold Th (S308: N), the image processing apparatus 20 determines whether the variable k has reached L (S310), and, when the variable k has not reached L (S310: N), the image processing apparatus 20 increments k (adds 1 to k) (S311), and returns to S306. Alternatively, when the variable k has reached L in S310 (S310: Y), the image processing apparatus 20 determines whether the variable j has reached M (S312).

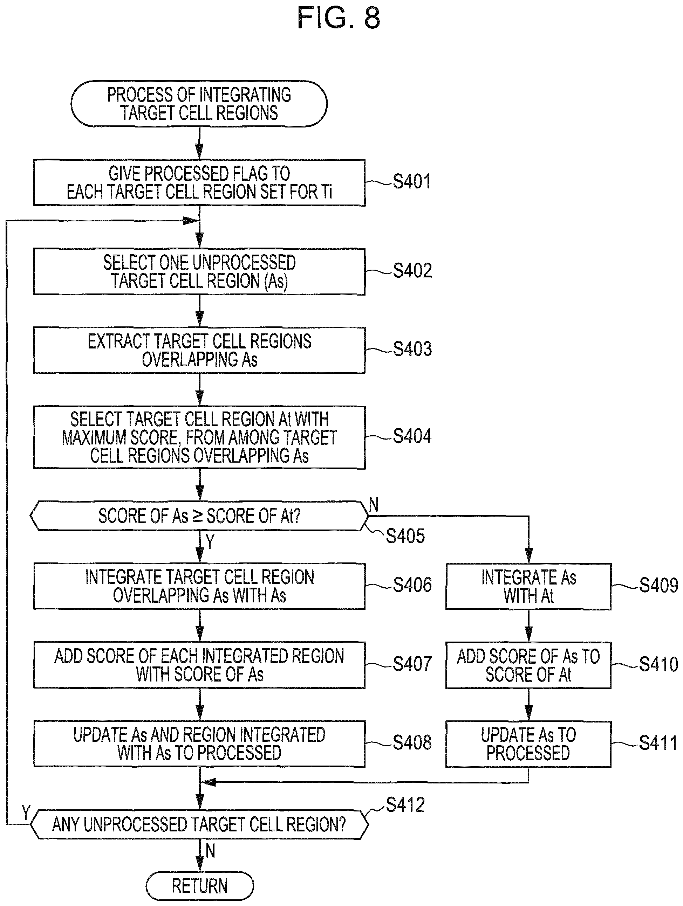

When the variable j has not reached M in S312 (S312: N), the image processing apparatus 20 increments j (adds 1 to j), initializes k to 1 (S313), and returns to S305. Alternatively, when the variable j has reached M in S312 (S312: Y), the image processing apparatus 20 executes a process of integrating target cell regions set for the sample Si (S314). Here, the process of integrating target cell regions will be described in detail on the basis of the flowchart illustrated in Fig. 8.

### [3-1-4. Process of Integrating Target Cell Regions]

As illustrated in Fig. 8, the image processing apparatus 20 gives a processed flag to each of target cell regions set for the captured image Ti of the sample Si (S401). Note that the processed flag is a true/false value. It is assumed that processed is true (T) and unprocessed is false (F), and the initial value of the processed flag given in S401 is false (F).

Next, the image processing apparatus 20 selects one target cell region whose processed flag value indicates unprocessed (S402). Hereinafter, the target cell region selected in S402 will be referred to as As.

The image processing apparatus 20 extracts other target cell regions overlapping the target cell region As selected in S402, from among the target cell regions set for the captured image Ti (S403). The image processing apparatus 20 selects a target cell region with the maximum score from among the target cell regions extracted in S403 (S404). Hereinafter, the target cell region selected in S404 will be referred to as At.

When the score of the target cell region As is greater than or equal to the score of the target cell region At (S405: Y), the image processing apparatus 20 integrates the target cell region extracted in S403 with the target cell region As (S406), and adds the score of this other target cell region being integrated to the score of the target cell region As (S407). For example, the target cell region integrated with the target cell region As may be deleted, or the state of the target cell region integrated with the target cell region As may be updated to integrated. The image processing apparatus 20 updates the processed flag of each of the target cell region As and the other target cell region integrated with the target cell region As to true (T) (S408).

Alternatively, when the score of the target cell region As is less than the score of the target cell region At (S405: N), the image processing apparatus 20 integrates the target cell region As with the target cell region At (S409), and adds the score of the target cell region As to the score of the target cell region At (S410). The image processing apparatus 20 updates the processed flag of the target cell region As to true (T) (S411).

When there remains an unprocessed target cell region after S408 or S411 (S412: Y), the image processing apparatus 20 returns to S402. When there remains no unprocessed target cell region (S412: N), the image processing apparatus 20 ends the process of integrating target cell regions, and returns.

Here, referring back to Fig. 7, a description will be continuously given. Having finished the process of integrating target cell regions set for the sample Si, when the variable i has not reached d (the number of detection target samples or the number of all samples) (S315: N), the image processing apparatus 20 increments i (adds 1), initializes j and k to 1 (S316), and returns to S302. Alternatively, when the variable i has reached d in S315 (S315: Y), the image processing apparatus 20 returns.

Alternatively, when the sample Si is not a detection target in S302 (S302: N) and the variable i has not reached d (S317: N), the image processing apparatus 20 increments i (adds 1), initializes j and k to 1 (S316), and returns to S302; and, when the variable i has reached d (S317: Y), the image processing apparatus 20 returns.

### [3-1-1(3). Main Process (3)]

Here, referring back to the flowchart in Fig. 5, a description will be continuously given. Having finished the process of detecting target cells in each specimen serving as a processing target, the image processing apparatus 20 outputs, on the basis of integrated target cell regions for each specimen, information on the target cell regions (S110), and ends the process. For example, for each specimen, the image processing apparatus 20 may sort the integrated target cell regions in descending order of the score of the target cell regions, and may display the sorted target cell regions on the display device 60.

According to the process according to the above-described first example, parameters for use in a detection process can be set on the basis of the estimated number of target cells included in a specimen serving as a processing target, and the difficulty in detecting target cells in the specimen serving as a processing target.

### [3-2. Second Example]

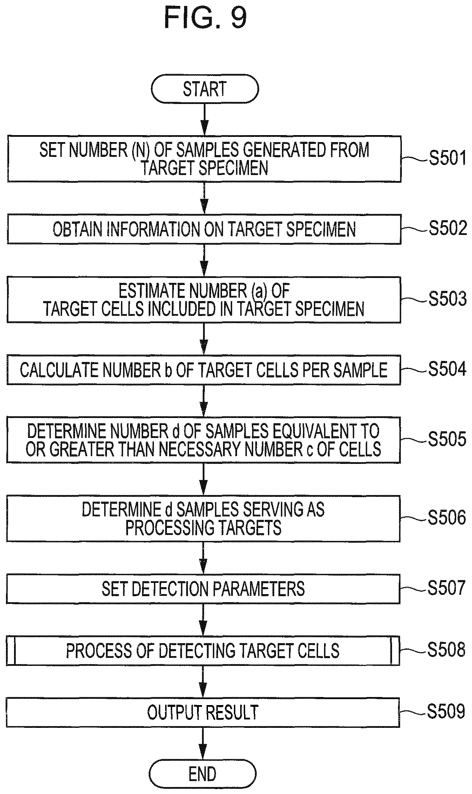

Next, on the basis of the flowchart illustrated in Fig. 9, a process according to a second example, executed by the image processing system 1, will be described in detail. The second example is different from the first example in the point that detection parameters are set without using the detection difficulty.

As illustrated in Fig. 9, on the basis of data input from the input device 50, the image processing apparatus 20 sets the number (N (N is an integer greater than or equal to 1)) of samples generated from a target specimen (S501), and obtains information on the target specimen (specimen information) (S502). Here, the specimen information may include at least one of information on the age, medical history, and weeks of pregnancy.

Next, on the basis of the specimen information obtained in S502, the image processing apparatus 20 estimates the number (a) of target cells included in the target specimen (S503). For example, the image processing apparatus 20 may identify a category to which the specimen information obtained in S502 belongs, and, on the basis of a representative number of target cells per unit blood, which is predetermined for the identified category, and the volume of blood of a preparation serving as a specimen, may calculate the estimated number of target cells included in the specimen.

On the basis of the number (a) of target cells, calculated in S503, and the number (N) of samples, set in S501, the image processing apparatus 20 calculates the number (b) of target cells per sample (S504). On the basis of the number (b) of target cells per sample, calculated in S504, the image processing apparatus 20 determines the number (d) of detection target samples equivalent to or greater than a necessary number (c) of cells needed for detection (S505).

Here, the image processing apparatus 20 selects and determines samples serving as processing targets, the number of which is the number (d) of detection target samples, determined in S505, from among a plurality of samples generated from the target specimen (S506).

Next, the image processing apparatus 20 sets detection parameters on the basis of information on each of the samples serving as processing targets, determined in S506, and the number (b) of target cells per sample, calculated in S504 (S507). For example, the detection parameters may include a detection target flag indicating whether the sample serves as a detection target, nucleus candidate region parameters indicating conditions of an image region extracted as a nucleus candidate region from the sample, and determination target region parameters indicating conditions for setting a determination target region set for an image of the sample. Here, the image processing apparatus 20 may set, for example, the detection target flag on the basis of the information on each sample serving as a processing target, determined in S506, and may set the nucleus candidate region parameters and the determination target region parameters on the basis of a margin (b·d-c) obtained by subtracting the necessary number (c) of cells from b·d, which is multiplication of the number (d) of detection target samples and the number (c) of target cells per sample.

On the basis of the detection parameters set in S507, the image processing apparatus 20 executes a process of detecting target cells in the specimen (S508). The process of detecting target cells is as illustrated in the flowchart illustrated in Fig. 7 and is common to the first example, a description of which will be omitted here.

Having finished the process of detecting target cells in each specimen serving as a processing target, the image processing apparatus 20 outputs, on the basis of integrated target cell regions for each specimen, information on the target cell regions (S509), and ends the process.

According to the process according to the above-described second example, parameters for use in a detection process can be set on the basis of the estimated number of target cells included in a specimen serving as a processing target.

### [3-3. Third Example]

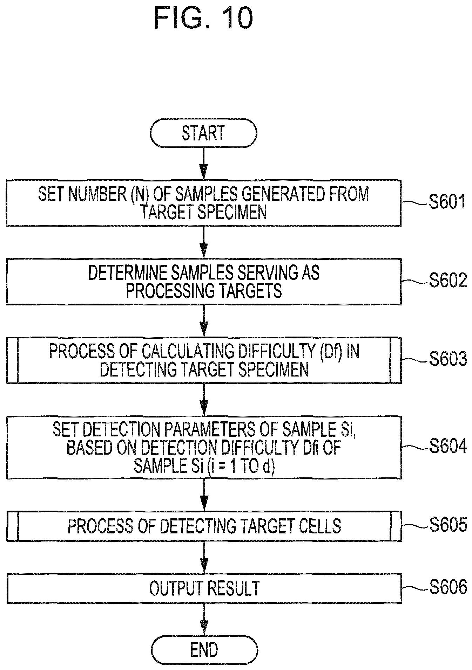

Next, on the basis of the flowchart illustrated in Fig. 10, a process according to a third example, executed by the image processing system 1, will be described in detail. The third example is different from the first example in the point that detection parameters are set without using the number of target cells, which is estimated for a target specimen.

As illustrated in Fig. 10, on the basis of data input from the input device 50, the image processing apparatus 20 sets the number (N (N is an integer greater than or equal to 1)) of samples generated from a target specimen (S601).

Here, the image processing apparatus 20 determines samples serving as processing targets, from among a plurality of samples generated from the target specimen (S602). For example, the image processing apparatus 20 may regard all the samples generated from the target specimen as processing targets, or may determine samples serving as processing targets on the basis of the detection difficulty Df calculated for the target specimen. For example, the image processing apparatus 20 may define a proportion in accordance with the range of the detection difficulty (specifically, the higher the detection difficulty, the higher the proportion), determines the number of samples serving as processing targets on the basis of the product of a proportion corresponding to the detection difficulty Df and the number of all the samples (specifically, the obtained product may be made an integer by rounding up), and determines samples serving as processing targets on the basis of the determined number of samples. Here, the number of samples serving as processing targets is represented as d.

Next, the image processing apparatus 20 executes a process of calculating the detection difficulty indicating the difficulty in detecting target cells in each sample serving as a processing target (S603). The process of detecting target cells is as illustrated in the flowchart illustrated in Fig. 6 and is common to the first example, a description of which will be omitted here.

Next, for the sample Si (i = 1 to d) serving as a processing target, the image processing apparatus 20 sets detection parameters of the sample Si on the basis of the detection difficulty Dfi calculated in S603. For example, the detection parameters may include a detection target flag indicating whether the sample serves as a detection target, nucleus candidate region parameters indicating conditions of an image region extracted as a nucleus candidate region from the sample, and determination target region parameters indicating conditions for setting a determination target region set for an image of the sample. Here, the image processing apparatus 20 may set, for example, the detection target flag on the basis of the information on each sample serving as a processing target, determined in S603, and may set the nucleus candidate region parameters and the determination target region parameters on the basis of the detection difficulty Dfi calculated for the sample Si.

On the basis of the detection parameters set in S604, the image processing apparatus 20 executes a process of detecting target cells in the specimen (S605). The process of detecting target cells is as illustrated in the flowchart illustrated in Fig. 7 and is common to the first example, a description of which will be omitted here.

Having finished the process of detecting target cells in each specimen serving as a processing target, the image processing apparatus 20 outputs, on the basis of integrated target cell regions for each specimen, information on the target cell regions (S606), and ends the process.

According to the process according to the above-described third example, parameters for use in a detection process can be set on the basis of the difficulty in detecting target cells in a specimen serving as a processing target.

The present invention is not construed as being limited to the above-described embodiment. For example, there is no problem in displaying target cell regions in order of score, without integrating the target cell regions. In addition, the image processing apparatus 20 is not construed as being limited to the case of obtaining a captured image of a specimen from the optical microscope 10, and the image processing apparatus 20 may obtain a captured image of a specimen from another computer.

## Claims

1. An image processing apparatus (20) comprising:
estimation means (23) configured to estimate based on a feature of a target specimen, a number of target cells included in the target specimen;
setting means (28) configured to set based on the estimated number of target cells, detection parameters regarding a process of detecting the target cells in the target specimen, wherein the detection parameters include information defining whether each of a plurality of samples generated from the target specimen serves as a detection target of detecting whether the target cells are included and information defining a condition for setting a determination target region for a captured image of a sample for determining whether target cells are included, and
determination means (30) configured to determine for a captured image of a sample serving as a detection target based on the detection parameters, whether the target cells are included in a determination target region set based on the detection parameters.

2. The image processing apparatus (20) according to Claim 1, further comprising
deciding means (27) configured to decide on a number of samples including target cells, a number of which is equivalent to or greater than a predetermined threshold which is a necessary number of target cells, based on the number of target cells, estimated by the estimation means (23), and a number of target cells per sample, calculated by dividing the estimated number of target cells for the target specimen by the number of samples generated from the target specimen, and
selection means (27) configured to select samples, a number of which is decided by the deciding means (27), from among the plurality of samples,
wherein the setting means (28) is configured to set the detection parameters based on information on the samples selected by the selection means.

3. The image processing apparatus (20) according to Claim 2,
wherein the setting means (28) is configured to set the setting condition such that more determination target regions are set as a difference between the predetermined threshold which is a necessary number of target cells, and a product of the number of samples, decided by the deciding means (27) and the calculated number of target cells per sample becomes smaller.

4. The image processing apparatus (20) according to any one of Claims 1 to 3, wherein the specimen is maternal blood and the apparatus further comprises:
detection difficulty calculating means (26) configured to calculate detection difficulty indicating difficulty in detecting a number of target cells included in the sample, based on a thickness of the sample, an intensity of the sample, and a proportion of white blood cells in the sample,
wherein the setting means (28) is configured to set the detection parameters based on the calculated detection difficulty.

5. The image processing apparatus (20) according to Claim 4,
wherein the setting means (28) is configured to set the setting condition such that more determination target regions are set as the detection difficulty becomes higher.

6. The image processing apparatus (20) according to Claim 4 or 5,
wherein the detection difficulty calculating means is configured to calculate the detection difficulty to be lower as thickness evenness of the sample becomes higher, to calculate the detection difficulty to be lower as an intensity of a nucleus included in the sample becomes greater, and to calculate the detection difficulty to be higher as a proportion of white blood cells included in the sample becomes higher.

7. The image processing apparatus (20) according to any one of Claims 1 to 6, further comprising:
integrating means (31) configured to integrate, in a case where there is a plurality of determination target regions that at least partially overlap each other and that are determined to include the target cells, the plurality of determination target regions with a determination target region determined by the determination means (30) to be most likely to include the target cells.

8. The image processing apparatus (20) according to any one of Claims 1 to 7,
wherein the specimen is maternal blood,
wherein the target cells are nucleated red blood cells,
wherein the feature of the specimen includes at least one of age, medical history, and weeks of pregnancy of a mother,
wherein a reference number of nucleated red blood cells per unit blood volume is defined for each category based on the feature of the specimen, and
wherein the estimation means (23) is configured to estimate a number of nucleated red blood cells included in the target specimen, based on a reference number of nucleated red blood cells, defined for a category in accordance with the feature of the target specimen, and a volume of blood of the target specimen.

9. An image processing method comprising:
an estimation step of estimating, based on a feature of a target specimen, a number of target cells included in the target specimen;
a setting step of setting, based on the estimated number of target cells, detection parameters regarding a process of detecting the target cells in the target specimen, wherein the detection parameters include information defining whether each of a plurality of samples generated from the target specimen serves as a detection target of detecting whether the target cells are included and information defining a condition for setting a determination target region for a captured image of a sample for determining whether target cells are included, and
a determining step of determining, for a captured image of a sample serving as a detection target based on the detection parameters, whether the target cells are included in a determination target region set based on the detection parameters.

10. A computer program comprising instructions which, when the program is executed by a computer, cause the computer to carry out the method of claim 9.

11. A computer-readable storage medium having stored thereon the computer program of claim 10.

## Patentansprüche

1. Bildverarbeitungsvorrichtung (20), umfassend:
eine Abschätzeinrichtung (23), konfiguriert zum Abschätzen einer Anzahl von Targetzellen in eine Targetprobe, basierend auf einem Merkmal der Targetprobe;
eine Einstelleinrichtung (28), konfiguriert, um basierend auf der abgeschätzten Anzahl von Targetzellen Nachweisparameter einzustellen bezüglich eines Prozesses des Nachweisens der Targetzellen in der Targetprobe, wobei die Nachweisparameter Information enthalten, welche definiert, ob jede aus einer Mehrzahl von aus der Targetprobe generierten Proben als ein Nachweistarget zum Nachweisen dient, ob die Targetzellen enthalten sind, außerdem Informationen enthalten, welche eine Bedingung für das Einstellen einer Bestimmungs-Targetzone für ein aufgenommenes Bild einer Probe definiert, um zu bestimmen, ob Targetzellen enthalten sind, und
eine Bestimmungseinrichtung (30), konfiguriert, um für ein aufgenommenes Bild der als Nachweistarget dienenden Probe basierend auf den Nachweisparametern zu bestimmen, ob die Targetzellen in einer Bestimmungs-Targetzone enthalten sind, die basierend auf den Nachweisparametern eingestellt sind.

2. Bildverarbeitungsvorrichtung (20) nach Anspruch 1, weiterhin umfassend:
eine Entscheidungseinrichtung (27), konfiguriert zum Entscheiden über eine Anzahl von Targetzellen enthaltenden Proben, eine Zahl, die äquivalent oder größer ist als ein vorbestimmter Schwellenwert, bei dem es sich um eine notwendige Anzahl von Targetzellen handelt, basierend auf der Anzahl von Targetzellen, die von der Abschätzeinrichtung (23) abgeschätzt wurden, und eine Anzahl von Targetzellen pro Probe, berechnet durch Dividieren der abgeschätzten Anzahl von Targetzellen für die Targetzone, durch die Anzahl von aus der Targetprobe erzeugten Proben, und
eine Auswahleinrichtung (27), konfiguriert, um unter den mehreren Proben solche Proben auszuwählen, deren Anzahl durch die Entscheidungseinrichtung (27) entschieden wurde,
wobei die Einstelleinrichtung (28) konfiguriert ist zum Einstellen der Nachweisparameter basierend auf Information über die von der Auswahleinrichtung ausgewählten Proben.

3. Bildverarbeitungsvorrichtung (20) nach Anspruch 2,
bei der die Einstelleinrichtung (28) konfiguriert ist zum Einstellen der Einstellbedingung derart, dass umso mehr Bestimmungs-Targetzonen eingestellt werden, desto geringer eine Differenz wird zwischen dem vorbestimmten Schwellenwert, der eine notwendige Anzahl von Targetzellen ist, und einem Produkt der Anzahl von Proben, die von der Entscheidungseinrichtung (27) entschieden wurden, und der berechneten Anzahl von Targetzellen pro Probe.

4. Bildverarbeitungsvorrichtung (20) nach einem der Ansprüche 1 bis 3, bei der die Probe mütterliches Blut ist und die Vorrichtung weiterhin aufweist:
eine Nachweisschwierigkeits-Berechnungseinrichtung (26), konfiguriert zum Berechnen einer Nachweisschwierigkeit, die eine Schwierigkeit angibt für das Nachweisen einer Anzahl von in der Probe enthaltenen Targetzellen, basierend auf einer Dicke der Probe, einer Intensität der Probe und einem Anteil weißer Blutkörperchen in der Probe,
wobei die Einstelleinrichtung (28) konfiguriert ist zum Einstellen der Nachweisparameter auf der Grundlage der berechneten Nachweisschwierigkeit.

5. Bildverarbeitungsvorrichtung (20) nach Anspruch 4,
bei der die Einstelleinrichtung (28) konfiguriert ist zum Einstellen der Einstellbedingung derart, dass je höher die Nachweisschwierigkeit wird, desto mehr Bestimmungs-Targetzonen eingestellt werden.

6. Bildverarbeitungsvorrichtung (20) nach Anspruch 4 oder 5,
bei der die Nachweisschwierigkeits-Berechnungseinrichtung konfiguriert ist zum Berechnen der Nachweisschwierigkeit derart, dass sie umso geringer ist, je höher die Dickengleichmäßigkeit der Probe ist, die Nachweisschwierigkeit so zu berechnen, dass sie geringer ist, wenn eine Intensität eines in der Probe enthaltenen Nukleus größer wird, und um die Nachweisschwierigkeit so zu berechnen, dass sie umso höher ist, je höher ein Verhältnis weißer Blutkörperchen in der Probe ist.

7. Bildverarbeitungsvorrichtung (20) nach einem der Ansprüche 1 bis 6, weiterhin umfassend:
eine Integriereinrichtung (31), konfiguriert, um dann, wenn es mehrere Bestimmungs-Targetzonen gibt, die einander teilweise überlappen, und die als Targetzellen enthaltend bestimmt werden, die mehreren Bestimmungs-Targetzonen mit einer Bestimmungs-Targetzone zu integrieren, welche von der Bestimmungseinrichtung (30) als diejenige bestimmt wurde, die am wahrscheinlichsten die Targetzellen enthält.

8. Bildverarbeitungsvorrichtung (20) nach einem der Ansprüche 1 bis 7,
bei der die Probe mütterliches Blut ist,
bei der die Targetzellen kemhaltige rote Blutkörperchen sind,
wobei das Merkmal der Probe mindestens eine der folgenden Größen enthält: Alter, medizinische Vorgeschichte und Schwangerschaftswochen einer Mutter,
wobei eine Referenzzahl von kemhaltigen roten Blutkörperchen pro Blutvolumen definiert ist für jede Kategorie, basierend auf dem Merkmal der Probe, und
wobei die Abschätzeinrichtung (23) konfiguriert ist zum Abschätzen einer Anzahl kemhaltiger roter Blutkörperchen in der Targetprobe, basierend auf einer Referenzzahl kemhaltiger roter Blutkörperchen, die definiert ist für eine Kategorie entsprechend dem Merkmal der Targetprobe, und einem Blutvolumen der Targetprobe.

9. Bildverarbeitungsverfahren, umfassend:
einen Abschätzschritt des Abschätzens einer Anzahl von in einer Targetprobe enthaltenen Targetzellen, basierend auf einem Merkmal einer Targetprobe;
ein Einstellschritt des Einstellens von Nachweisparametern, basierend auf der abgeschätzten Anzahl von Targetzellen, wobei die Nachweisparameter einen Prozess des Nachweisens der Targetzellen in der Targetprobe betreffen, wobei die Nachweisparameter Information enthalten, die definiert, ob jede der aus der Targetproben generierten Proben als ein Nachweistarget des Nachweisens dient, ob die Targetzellen enthalten sind, und Information enthalten, welche eine Bedingung zum Einstellen einer Bestimmungs-Targetzone für ein aufgenommenes Bild einer Probe zum Bestimmen, ob Targetzellen enthalten sind, definiert, und
einen Bestimmungsschritt des Bestimmens, der für ein aufgenommenes Bild einer als Nachweistarget basierend auf den Nachweisparametern dienenden Probe zu bestimmen, ob die Targetzellen in einer Bestimmungs-Targetzone enthalten sind, basierend auf den Nachweisparametern.

10. Computerprogramm mit Befehlen, die bei Ausführung des Programms durch einen Computer diesen veranlassen, das Verfahren nach Anspruch 9 auszuführen.

11. Computerlesbares Speichermedium, auf dem das Computerprogramm nach Anspruch 10 gespeichert ist.

## Revendications

1. Appareil de traitement d'image (20), comprenant :
un moyen d'estimation (23) configuré pour estimer, sur la base d'une caractéristique d'un spécimen cible, un nombre de cellules cibles incluses dans le spécimen cible ;
un moyen de réglage (28) configuré pour régler, sur la base du nombre estimé de cellules cibles, des paramètres de détection concernant un processus de détection des cellules cibles dans le spécimen cible, dans lequel les paramètres de détection incluent des informations définissant si oui ou non chacun d'une pluralité d'échantillons générés à partir du spécimen cible sert de cible de détection quant à la détection de si oui ou non les cellules cibles sont incluses, et des informations définissant une condition pour régler une région cible de détermination pour une image capturée d'un échantillon pour déterminer si oui ou non des cellules cibles sont incluses, et
un moyen de détermination (30) configuré pour déterminer, pour une image capturée d'un échantillon servant de cible de détection, sur la base des paramètres de détection, si oui ou non les cellules cibles sont incluses dans une région cible de détermination réglée sur la base des paramètres de détection.

2. Appareil de traitement d'image (20) selon la revendication 1, comprenant en outre :
un moyen de décision (27) configuré pour décider quant à un nombre d'échantillons incluant des cellules cibles, dont le nombre est supérieur ou équivalent à un seuil prédéterminé, lequel est un nombre nécessaire de cellules cibles, sur la base du nombre de cellules cibles, estimé par le moyen d'estimation (23), et à un nombre de cellules cibles par échantillon, calculé en divisant le nombre estimé de cellules cibles pour le spécimen cible par le nombre d'échantillons générés à partir du spécimen cible, et
un moyen de sélection (27), configuré pour sélectionner des échantillons, dont un nombre est décidé par le moyen de décision (27), à partir de la pluralité d'échantillons,
dans lequel le moyen de réglage (28) est configuré pour régler les paramètres de détection sur la base des informations sur les échantillons sélectionnés par le moyen de sélection.

3. Appareil de traitement d'image (20) selon la revendication 2,
dans lequel le moyen de réglage (28) est configuré pour régler la condition de réglage de telle sorte que plus de régions cibles de détermination soient réglées sous la forme d'une différence entre le seuil prédéterminé, lequel est un nombre nécessaire de cellules cibles, et un produit du nombre d'échantillons, décidé par le moyen de décision (27), et que le nombre calculé de cellules cibles par échantillon diminue.

4. Appareil de traitement d'image (20) selon l'une quelconque des revendications 1 à 3,
dans lequel le spécimen est du sang maternel, et l'appareil comprend en outre :
un moyen de calcul de difficulté de détection (26) configuré pour calculer une difficulté de détection, indiquant une difficulté à détecter un nombre de cellules cibles incluses dans l'échantillon, sur la base d'une épaisseur de l'échantillon, d'une intensité de l'échantillon, et d'une proportion de globules blancs dans l'échantillon, et
dans lequel le moyen de réglage (28) est configuré pour régler les paramètres de détection sur la base de la difficulté de détection calculée.

5. Appareil de traitement d'image (20) selon la revendication 4,
dans lequel le moyen de réglage (28) est configuré pour régler la condition de réglage de telle sorte que plus de régions cibles de détermination soient réglées à mesure que la difficulté de détection augmente.

6. Appareil de traitement d'image (20) selon la revendication 4 ou 5,
dans lequel le moyen de calcul de difficulté de détection est configuré pour calculer la difficulté de détection pour que celle-ci diminue à mesure que l'uniformité d'épaisseur de l'échantillon augmente, pour calculer la difficulté de détection pour que celle-ci diminue à mesure qu'une intensité d'un noyau inclus dans l'échantillon augmente, et pour calculer la difficulté de détection pour que celle-ci augmente à mesure que la proportion de globules blancs inclus dans l'échantillon augmente.

7. Appareil de traitement d'image (20) selon l'une quelconque des revendications 1 à 6, comprenant en outre :
un moyen d'intégration (31) configuré pour intégrer, dans un cas où il existe une pluralité de régions cibles de détermination, lesquelles présentent un chevauchement au moins partiel entre elles, et lesquelles sont déterminées de manière à inclure les cellules cibles, la pluralité de régions cibles de détermination présentant une région cible de détermination déterminée par le moyen de détermination (30) devant très probablement inclure les cellules cibles.

8. Appareil de traitement d'image (20) selon l'une quelconque des revendications 1 à 7,
dans lequel le spécimen est du sang maternel ;
dans lequel les cellules cibles sont des globules rouges nucléés ;
dans lequel la caractéristique du spécimen inclut au moins un élément parmi l'âge, les antécédents médicaux, et les semaines de grossesse d'une mère ;
dans lequel un numéro de référence de globules rouges nucléés par unité de volume sanguin est défini pour chaque catégorie sur la base de la caractéristique du spécimen, et
dans lequel le moyen d'estimation (23) est configuré pour estimer un nombre de globules rouges nucléés inclus dans le spécimen cible, sur la base d'un numéro de référence de globules rouges nucléés, défini pour une catégorie conformément à la caractéristique du spécimen cible, et d'un volume de sang du spécimen cible.

9. Procédé de traitement d'image, comprenant les étapes suivantes :
une étape d'estimation pour estimer, sur la base d'une caractéristique d'un spécimen cible, un nombre de cellules cibles incluses dans le spécimen cible ;
une étape de réglage pour régler, sur la base du nombre estimé de cellules cibles, des paramètres de détection concernant un processus de détection des cellules cibles dans le spécimen cible, dans lequel les paramètres de détection incluent des informations définissant si oui ou non une pluralité d'échantillons générés à partir du spécimen cible sert de cible de détection quant à la détection de si oui ou non les cellules cibles sont incluses, et des informations définissant une condition pour régler une région cible de détermination pour une image capturée d'un échantillon pour déterminer si oui ou non des cellules cibles sont incluses, et
une étape de détermination pour déterminer, pour une image capturée d'un échantillon servant de cible de détection, sur la base des paramètres de détection, si oui ou non les cellules cibles sont incluses dans une région cible de détermination réglée sur la base des paramètres de détection.

10. Programme informatique comprenant des instructions, lesquelles lorsque le programme est exécuté par un ordinateur, font en sorte qu'un ordinateur mette en œuvre le procédé selon la revendication 9.

11. Support de stockage lisible par ordinateur sur lequel est stocké le programme informatique selon la revendication 10.
